(19) Europäisches Patentamt
European Patent Office
Office européen des brevets

(11) EP 2 017 609 A1

(12) **EUROPEAN PATENT APPLICATION**

(43) Date of publication:
**21.01.2009 Bulletin 2009/04**

(51) Int Cl.:
***G01N 27/414*** *(2006.01)*

(21) Application number: **07014037.1**

(22) Date of filing: **18.07.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicants:
- **Université Catholique De Louvain**
  **1348 Louvain-La-Neuve (BE)**
- **Université Libre de Bruxelles**
  **1050 Brussels (BE)**

(72) Inventors:
- **Demoustier-Champagne, Sophie**
  **B-5032 Bossière (BE)**
- **Jonas, Alain**
  **B-1348 Louvain-la-Neuve (BE)**
- **Bayot, Vincent**
  **B-1348 Louvain-la-Neuve (BE)**
- **Nuysten, Bernard**
  **B-5030 Sauvenière (BE)**
- **Blondeau, Françoise**
  **B-1348 Louvain-la-Neuve (BE)**
- **Demeersman, Benoît**
  **B-1400 Nivelles (BE)**
- **Tang, Xiaohui**
  **B-1348 Louvain-la-Neuve (BE)**
- **Godfriod, Edmond**
  **B-1120 Bruxelles (BE)**
- **Jaquet, Alain**
  **B-1160 Bruxelles (BE)**
- **Prevot, Pierre-Paul**
  **B-1040 Bruxelles (BE)**

(74) Representative: **Bird, William Edward et al**
**Bird Goën & Co.**
**Klein Dalenstraat 42A**
**3020 Winksele (BE)**

(54) **Method and device for high sensitivity and quantitative detection of chemical/biological molecules**

(57) A sensor for detecting the presence of predetermined chemical/biological molecules, the sensor having a MOSFET device (2,3,6,7,9,10,12,13), the gate having an array of nanoscale fingers (6,7), and on fingers or in spaces between the fingers, chemical/biological receptor molecules (15) suitable for selectively binding with the predetermined chemical/biological molecules, the sensor also having circuitry (4,5,11,12 ) for measuring a change in a change in drain current of the FET caused by the predetermined chemical/biological molecules becoming bound to the receptor molecules. Sensitivity and reliability can be increased compared to capacitive sensors.

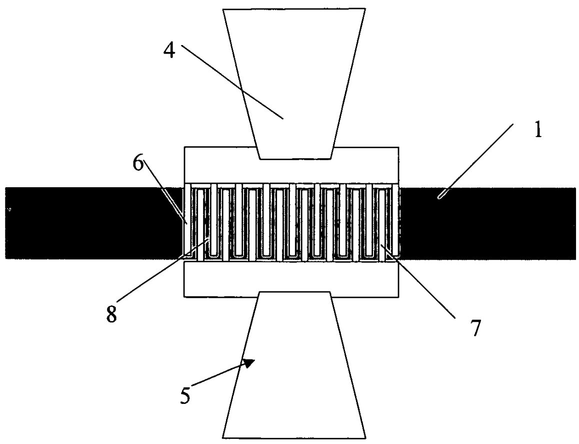


FIG. 2

EP 2 017 609 A1

## Description

### FIELD OF THE INVENTION

The present invention relates to a sensor for detecting chemical/biological molecules, and to corresponding methods of manufacturing, preparing and using such sensors. Particularly, it relates to a sensor for the electrical detection of the presence in a fluid such as a buffered solution of bio-macromolecules such as proteins, which bind to complementary molecular or bio-molecular probes grafted onto a nanoscale finger-like (interdigitated or not) electrode array, which is the gate of a metal oxide semiconductor field effect transistor (MOSFET), hereafter called the IDA-gate MOSFET sensor.

### BACKGROUND OF THE INVENTION

There are many different methods to detect the presence of chemical/biological molecules. They can be divided into two main general categories: optical detection and electric detection. Typical optical detection methods include enzyme-linked immunosorbent assay, surface plasma resonance, localized surface plasma resonance spectroscopy, surface-enhanced raman scattering spectroscopy, fluorescent, radioscopy methods, optical fiber-based sensors and other optical nanosensors (such as nanoparticle labeling assay and quantum dot nanosensor based on fluorescence resonance energy transfer).

In electric detection methods, the ion sensitive field-effect transistor (ISFET) has been the subject of marked attention since it was first reported by P. Bergveld in 1970. In this device, the metal gate of a MOSFET is removed, leaving the bare oxide as the actual contact between the electronic system and the chemical environment (electrolyte). When chemical ions are adsorbed on the electrolyte/oxide interface, the threshold voltage of the MOSFET is modified by the electrolyte/oxide interface potential. Therefore, the presence of chemical ions can be monitored by measuring the change in the threshold voltage. In 1978, Schenck proposed to develop a protein sensor based on the operational mechanism of the ISFET. It was expected that the binding of protein molecules to the gate in the FET structure would modulate the threshold voltage. Later, it was found that such an operational mechanism cannot work due to the fact that counter ions in an aqueous solution shield the charged protein molecules. Therefore, P. Bergveld et al. introduced a stimulus-response measurement. The measurement was carried out in a flow-through system: when a stimulus electrolyte with an intense ion concentration was injected into a streaming electrolyte with the same pH as the former, the protein molecules and the gate surface of ISFET would immediately respond to this change in ion concentration. Although this measurement has the ability to detect protein molecules, it is difficult to separate the mixed responses from protein molecules and interfering ions. Recently, Sergio Bermejo et al. proposed to use blind source separation techniques and independent component analysis to separate the main detected ions and interfering ions ( "ISFET source separation: foundations and techniques", Sensors and Actuators 2006 B, 113, 222). But the on-line applicability of this method is limited by pre-calibration and normalization steps.

Actually, the threshold voltage can also be modified by changing the work function of the gate material through a chemical reaction from outside. But, a counter electrode is then needed to prevent an ongoing reaction (P. Bergveld, Sensors and Actuators B 2003, 88 1). On the other hand, the threshold voltage can also be modified by changing the gate oxide capacitance. This means that the gate oxide of a MOSFET can be replaced by an organic insulating layer, e.g. with a hydrogel serving as a matrix for the immobilisation of charged molecules. However, critical challenges for ISFET application and development are (1) they need large sample volumes in the measurement; (2) they present small signal/noise ratio and low sensitivity; (3) their measurement procedure are complex, such as stimulus-response measurement; (4) they are limited by the choice of gate materials; (5) their encapsulation is difficult due to the fact that the measurement is carried out in an aqueous solution; (6) and most importantly they need to have a reference electrode, which is difficult to integrate in the device.

The electrical sensor research has been progressing with the development of nanotechnologies. Cui et al. proposed in Science 2001, 293, 1289, a nanowire sensor, which is a MOSFET nanowire transistor (the nanowire connects source and drain) without a metal gate. When a layer of chemical/biological ions attaches on the surface of the nanowire, the drain current shifts upward or downward. The one-dimensional morphology of the nanowire is expected to increase sensor sensitivity up to the single-molecule detection level.

Moreover Talin et al. reported in the article "Large area, dense silicon nanowire array chemical sensors", Applied Physics Letters 2006, 89, 153102, a nanowire sensor array. At the same time, Fernado et al., also reported in the paper "Fabrication of silicon nanowire devices for ultrasensitive, label-free, real-time detection of biological and chemical species" Nature Protocols 2006, 1 (4), 1711, another nanowire sensor array. These arrays integrate many individual sensors on one chip. They show opportunity for selective multiplexed detection of chemical/biological species. But since detections are carried out in a solution, it is necessary to integrate a microfluidic system, which makes the fabrication of such chips more complex and expensive.

Interdigitated electrode arrays (IDA) have been studied and employed as sensors for several years. Ehret et

al. designed in paper entitled "Monitoring of cellular behavior by impedance measurements on interdigitated electrode structures" (Biosensors and Bioelectronics 1997, 12, 29) a micro-scale IDA to obtain the impedance data attributable to cell attachment. Peter et al. in the article "Nanoscale interdigitated electrode arrays for biochemical sensors" (Sensors and Actuators B, 1998, 49, 73) performed theoretical calculations on a nano-scale IDA and compared the results with experimental data. The immobilization of glucose oxidase and bulk-insensitive behavior has been monitored by measuring the capacitance/resistance change between two combs. A major limitation related to the use of IDA alone concerns the sensitivity since the method can only detect large electrical signal variation and high ion concentrations. Moreover, any conduction path (for example a nanoscale dot) between two combs can result in false readings.

Flandre et al. invented in United Sates Patent Application Publication 2005, US2005/0227373 A1, a method for capacitive detection of the presence of DNA. In order to increase the electrical signal variation, a DNA sample, bonded on a non-conductive surface of micron-scale interdigitated electrode array, is directly or indirectly labeled with a conductive label, for example as obtained by silver precipitation. Although this method demonstrates a large sensitivity (about 1: 1000000), it is a label-dependent detection method.

## SUMMARY OF THE INVENTION

The present invention is based on appreciating that (1) the electrical signal variation is not so large in the known IDA capacitive/resistive sensors and it is necessary to integrate a signal amplifier to obtain a high sensitivity in such known sensors; and (2) the reliability is not so high in the IDA capacitive/resistive sensors, since any conduction path (for example a nanoscale dot) between two combs can result in false readings.

The above discussed problems may be reduced or overcome by a sensor according to embodiments of the present invention.

An object of some embodiments of the present invention is to provide a sensor for detecting the presence of chemical/biological molecules, with a sensitivity better than 1 ng/ml.

It is a further object of some embodiments of the present invention to provide a sensor for detecting the presence of chemical/biological molecules by means of label-free detection with small sample volumes about 1 μl.

It is another object of some embodiments of the present invention to provide a sensor for detecting the presence of chemical/biological molecules by rapid and direct analysis.

It is yet another object of some embodiments of the present invention to provide a method and a device for detecting the presence of chemical/biological molecules which is especially easy to miniature and to be compatible with CMOS technology.

A first aspect of the present invention provides a sensor for detecting the presence of chemical/biological molecules, the sensor being an MOSFET device, the gate of MOSFET device having at least one electrode in the form of an array of fingers, and in spaces between or on the fingers, the sensor having chemical/biological receptor molecules, the sensor also having circuitry for sensing a change in electrical characteristics of the MOSFET caused by the chemical/biological molecules becoming bound to the receptor molecules. The chemical/biological molecules can be predetermined molecules or may contain a predetermined motif.

A notable consequence of having such an array of fingers is that sensitivity can be increased compared to capacitive sensors. Furthermore the reliability can be increased since a short circuit between fingers may leave other fingers unaffected and so will have less effect on the sensing than a short circuit between plates of a capacitor type sensor for example.

The fingers can be in any shape including straight or curved fingers, elongated or not, the configuration of fingers can be interdigitated or not, the fingers can be in varying thickness which can provide effective spaces to enable the molecules in the spaces to alter the field strength in the channel

Another aspect provides a corresponding method of preparing such a sensor. Another aspect provides a method of using the sensor. Another provides a method of manufacturing such a sensor, or fabricating and characterizing such a sensor.

Embodiments of the apparatus or the method can have any additional features, some of which are set out in the claims, and described in more detail below. One such additional feature is the MOSFET comprising an IDA-gate.

Another is the step of binding a linker on the surface of the IDA-gate or in the space between fingers for the attachment of chemical/biological molecules.

Another is the step of attaching chemical/biological receptor molecules on the linker already bound onto the surface of the IDA-gate.

Before using the sensor, a preliminary step is sensing the presence of the attached chemical/biological receptor molecules on the linker by measuring the change in subthreshold characteristics of the IDA-gate MOSFET.

The use of the sensor for sensing can then result in anchoring or binding of the target chemical/biological molecule being sensed, to its complement, the receptor already attached on the linker.

Then the circuitry can be used for sensing of the presence of the target chemical/biological molecule bound

with its complement, the receptor molecules, by measuring a change in subthreshold characteristics of the IDA-gate MOSFET.

Another such additional step is measuring, before the binding of the linker, of the subthreshold characteristics. The preliminary subthreshold characteristics may be compared with the subthreshold characteristics during the sensing steps.

A further additional step can be a step of physical characterization to observe the presence of chemical/biological molecules onto the surface of IDA-gate. Any method of surface characterization which is sensitive to the anchored species can be used, such as AFM (atomic force microscopy), SEM (scanning electron microscopy) and AFM in the single molecule force spectroscopy mode.

Another such additional feature is apparatus or steps for providing in parallel a capacitive/resistive sensor device for determining the presence of chemical/biological molecules, when two combs are used for the IDA. Indeed, the IDA-gate itself is a capacitive/ resistive sensor, which can be used to detect the change in capacitance/resistance between combs before and after the presence of chemical/biological molecules.

According to other additional features, the IDA-gate MOSFET can work either in inversion or accumulation mode. The starting substrate of the IDA-gate MOSFET can consist of a P-type, N-type and intrinsic semiconductor, usually but not necessarily silicon. The source/drain region of the IDA-gate MOSFET can be formed by implantation or silicidation process. The insulating layer between the IDA-gate and the channel can be oxide, nitride, high-k materials or any low-conductive (preferably dielectric) materials (organic or inorganic). The IDA-gate can be made in any conductive materials, such as gold, platinum even a low cost metal, such as aluminium, or any conducting material (organic or inorganic).

For a given gate voltage, drain current value of IDA-gate MOSFET changes when detecting the presence of chemical/biological molecules onto the surface of the IDA-gate at least when the infection of the target chemical/biological molecule with its complement occurs.

The nanoscale array of fingers forming the gate may be perpendicular or parallel to the channel (or any other orientation), but perpendicular orientation usually provides larger current changes. The array can be composed of one comb or two combs. The finger spacing and width of the comb can be different or identical. The shape of the fingers can be varied, although lines will usually be preferred. While it would kill an IDA capacitive/resistive detector, it is not a problem for the IDA-gate MOSFET if even a nanoscale contact remains located as an unwanted short circuit between the two combs. The nanoscale array can be fabricated by E-beam lithography or a low cost method, such as nanoimprint, or any other method of lithography with sufficient resolution.

A back gate can be provided for the IDA-gate MOSFET to ground during the measurement. The back gate also can be used to verify the performance of conventional MOSFET, or most importantly to tune the detection regime of the nano-gate by shifting the threshold voltage.

Another such additional feature of some embodiments of the present invention is a reference device such as a second IDA-gate MOSFET, not exposed to the target molecules, enable a comparison of the current change with and without the presence of chemical/biological molecules.

In principle, the sensor can be in the form of connections to external measurement equipment for example

The term "IDA-gate MOSFET" refers to the device in which the blanket metal/polysilicon gate of the conventional MOSFET is replaced by a finger-like (optionally interdigitated) nanoscale electrode array. In fact, this device can be a capacitive/resistive sensor coupled with a field effect transistor. It is a capacitive sensor in the sense that the anchoring of molecular species modifies the gate channel capacitance, which is amplified by the field effect of the transistor and detected by a change in channel current.

It is sensitive for detecting the binding of chemical/biological molecules onto the surface of the IDA-gate. It is also sensitive for sensing the infection of chemical/biological molecules with its complement onto the surface of IDA-gate.

These and other features and advantages of the present invention will be described in detail as follows, with the accompanying drawings, which illustrate the principle of the present invention.

## BRIEF DESCRIPTION OF THE DRAWINGS

FIG. 1 shows a schematic top view of an IDA-gate MOSFET sensor device without the IDA-gate according to an embodiment of the present invention.

FIG. 2 shows a schematic top view of an IDA-gate with two combs according to an embodiment of the present invention.

FIG. 3 shows a schematic top view of an IDA-gate with one comb according to an embodiment of the present invention.

FIG. 4 shows a schematic cross-sectional view (a part of) of an IDA-gate MOSFET sensor device according to an embodiment of the present invention.

FIG. 5 shows a schematic total architecture of an IDA-gate MOSFET with four connecting pads according to

an embodiment of the present invention.

FIG. 6 shows a schematic cross-sectional view (a part of) of an IDA-gate MOSFET sensor device with a back gate underneath a non-conductive layer according to an embodiment of the present invention.

FIG. 7A, 7B, 7C show details of the binding of the linker onto the surface of IDA-gate, the attachment of chemical/biological receptor molecules on the linker, and the anchoring of specific chemical/biological analyte molecule with its complement according to an embodiment of the present invention.

FIG. 8 shows an equivalent circuit of the channel in an IDA-gate MOSFET sensor device according to an embodiment of the present invention before binding the linker, chemical/biological receptor molecules and target chemical/biological molecules.

FIG. 9 shows an equivalent circuit of a bare IDA-gate MOSFET sensor device according to an embodiment of the present invention.

FIG. 10A, 10B show a cross-section of two fingers and equivalent circuit of capacitance in a bare IDA-gate MOSFET sensor device according to an embodiment of the present invention.

FIG. 11 shows a graph of Id(Vg) characteristics of an IDA-gate MOSFET sensor for the bare device; the binding of thiol linker; the attachment of an antigen to the linker and the anchoring of a specific antibody to its complement. For the sake of demonstration, the antigen IRIS and the antibody anti-IRIS are selected for this figure; however, any complementary pair of bio-macromolecules or of molecules could have been selected as well.

FIG. 12A, 12B are SEM top views of an IDA-gate covered by anti-IRIS with concentrations of 0.7 ng/ml and 360 ng/ml, respectively.

FIG. 13 is a graph showing the sensitivity of an IDA-gate MOSFET sensor as a function of anti-IRIS concentrations for detecting the infection of IRIS and anti-IRIS.

## DETAILED DESCRIPTION

Embodiments of the present invention will be described in detail with respect to certain drawings but the invention is not limited thereto. For illustrative purposes, most of the drawings are schematic, therefore, the size of the elements are not drawn to scale. Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

The term "comprising", used in the claims, should not be interpreted as being restricted to the means listed thereafter; it does not exclude other elements or steps. Thus, the scope of the expression "a device comprising means A and B" should not be limited to devices consisting only of components A and B. It means that with respect to the present invention, the only relevant components of the device are A and B.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein.

Embodiments of the present invention will mainly be described with reference to detecting the infection of antigen (IRIS) and antibody (anti-IRIS) but are not limited to them and the present invention can be applied in wider chemical and biological fields.

Nanostructures open new perspectives for the development of electrical sensors. The introduction of the ID A-gate MOSFET sensor could revolutionize many aspects of biological/chemical detection. Applications are, for example, biotechnology (e. g., the analysis of protein, DNA, RNA, enzyme and molecular components of bacteria), medical diagnostics (e. g., AIDS, cancer, bio- or immuno-sensors), drug screening, pharmacological research, food safety and environmental protection.

The IDA-gate MOSFET sensor is a MOSFET device except for the fact that the usual blanket metal/polysilicon gate is replaced by a nanoscale finger-like (interdigitated or not) electrode array. In the case of the interdigitated nanoelectrode device, when a bias is applied to one of the combs in the array while the other is floating, it is analogous to applying a periodic gate voltage to the MOSFET device. The current drive is lowered in the IDA-gate MOSFET compared to a conventional MOSFET due to the presence of different potential, resulting in a higher resistance region, underneath the floating comb and the spacing. When a solution containing the chemical/biological molecules to be detected is dropped on the array surface where complementary receptor molecules were bound through an appropriate linker, an additional layer of chemical/biological molecules from the drop forms on the receptor molecules. Hence, the periodic electrical field is modified, which results in a change of current drive in the MOSFET. For a given gate voltage, the drain current is a function of the chemical/biological molecular layer thickness and molecular concentration. For example, if

an antigen is taken as receptor molecule and is bound on the array surface by an appropriate binding method as, e.g., by the reaction of one of its amine groups to a carboxylic-ended thiol assembled on the electrodes, the presence of the complementary antibody in the dropped solution will result in the formation of a supplementary antibody layer on the array surface, resulting in a enhancement in drain current. Embodiments of the present invention thus can electrically detect chemical/biological molecules. It also offers unique opportunities for selective multiplexed detection of chemical/biological molecules since a large number of sensors can be integrated on one chip.

The IDA-gate MOSFET sensors presented here have the advantages of label-free and direct analysis, small sample volumes, scaling possibility, good reproducibility and compatibility with CMOS technology. Potential applications are not only in hospitals and specialised laboratories but also in the form of portable devices.

One method of fabrication of IDA-gate MOSFET sensor may be as follows: first a starting substrate is provided, the substrate may be a semiconductor such as silicon, germanium, silicon germanium (SiGe) or any other organic or inorganic semiconductor. It may also be a commercially available SOI (Silicon on Insulator) wafer. The description is given for a silicon wafer. Then a pad oxide may be grown or deposited on the substrate in order to protect the substrate surface from implantation damage. The substrate was doped by ion implantation, according to the desired MOSFET operational mode.

A device layout (**FIG. 1**) having a narrow channel **1** connected by larger source **2** and drain **3** regions is transferred to the substrate using the current optical lithography procedures. Reactive ion etching (RIE) may be used to etch the oxide and the substrate. For an SOI wafer, the silicon overlay may be etched down to the buried oxide to avoid the neighboring devices from interconnection. The source/drain region is defined and is highly doped by ion implantation. The dopants may be N-type, P-type, according to the desired operational mode. For example, P-channel inversion MOSFET requires N-type dopant, while N-channel inversion MOSFET requires P-type dopant. Contrarily, P-channel and N-channel accumulation MOSFET require P-type and N-type dopants, respectively.

The accumulation mode MOSFET may be suitable to detect the presence of chemical/biological molecules. Because the detection only depends on the relative change in drain current, it does not matter even if there is a little current (normally-on) at the off-state of the MOSFET. One way to reduce the cost can be to replace the expensive implantation step by a cheap silicidation process for a source/drain architecture. For example, platinum and iridium can be used for P-type silicon contacts as well as erbium and ytterbium for N-type silicon contacts.

The oxide damaged by implantation is removed. A high quality insulating layer may be grown or deposited on the surface of the device. Also the high quality insulating layer may isolate the sidewall of the device. The thickness of the insulating layer is chosen as thin as possible to obtain a high sensitivity, but avoiding a too large gate current leakage. The high quality insulating layer may be a $SiO_2$, $Si_3N_4$ or high-k materials, or any other dielectric layer, non-conductive organic or inorganic, providing the desired surface characteristics, e. g. not being chemically etched during the binding of the linker, the attachment of chemical/biological receptor molecules and the infection of chemical/biological molecule with its complement.

Then the layout **4, 5 (FIG. 2)** of the nanoscale electrode array gate is transferred to resist layer (possibly PMMA for example) by a high resolution lithography tool. This tool may be E-beam lithography or a low cost method, such as nanoimprint. The finger width **6, 7** and spacing **8** are chosen as narrow as possible in order to obtain the highest sensitivity, while maintaining spacing that is still sufficient to be compatible with the size of chemical/biological molecules. The finger width **6, 7** and spacing **8** may be identical or different. Preferably, the finger width is in a range between 10 nanometer and 100 micron, more preferably between 10 nanometer and 1 micron, and most preferably between 10 nanometer and 200 nanometer. The finger spacing is comprised between 10 nanometer and 100 micron, preferably between 10 nanometer and 1 micron and most preferably between 10 nanometer and 100 nanometer. The detection surface of the array is chosen as small as possible to miniaturize the size of the devices, but still within the limit of the measurements.

A layer of metal, easily binding the linker, is deposited on the resist layer (possibly PMMA) for a lift-off process. Next the nanoscale electrode array is formed. The nanoscale electrode array should not be chemically etched during the binding of the linker, the attachment of chemical/biological receptor molecules and the infection of chemical/biological molecule with its complement. For materials which would be etched and damaged easily, a passivation layer may be added. Thus, the nanoscale electrode array can be made of gold, platinum, silver, ruthenium, iridium, palladium, even low cost conductor materials, such as aluminium, copper, iron, zirconium or any appropriate conductive material (organic or inorganic) that enables anchoring of the desired species. Preferably, the nanoscale electrodes are made of gold, platinum, silver. More preferably, the nanoscale electrodes are made of gold.

An advantage of the nanoscale electrode array gate is that the gate itself can be used as a capacitive/resistive sensor to measure capacitance/resistance change before and after the presence of chemical/biological molecules. The measurement can be carried out directly between both combs **4, 5 (FIG.2)** or a one-comb gate MOSFET sensor device **(FIG. 3)** according to the present invention. The one-comb gate MOSFET sensor device is well adapted to miniaturisation of the device, because the number of gate electrodes is reduced from two **4, 5 (FIG.2)** to one **5 (FIG. 3).**

Alternatively, the linker and receptor molecules may be attached to other surfaces facing the inter-finger spacing. Still another appropriate method may be used to attach the linker and receptor molecules to both the inter-finger spacing

and the finger surface.

The thickness of the nanoscale electrode array **6,7 (FIG. 4)** is chosen as thick as possible to increase the sidewall surface of fingers, providing more sites for the binding of the linker, but still compatible with the lift-off process. Preferably, the thickness of the nanoscale electrode is comprised between 5 nanometers and 100 nanometers, more preferably between 5 nanometers and 50 nanometers and most preferably between 5 nanometers and 25 nanometers.

To improve the adhesion between the insulating layer **9 (FIG.4)** and the nanoscale electrode **6,7 (FIG. 4),** an intermediate layer may be added. Typically, this intermediate layer is made of titanium.

Windows **11 (FIG. 5)** may be opened in source/drain regions for the deposition of metal pads, which are used to connect the external electrodes by any method that realizes bonding of wires. There are two source and two drain connections in the actual device for reliability reason. There can be any number of connections with a minimum of one for the source and one for the drain.

Finally, the back gate **12 (FIG. 6)** may be realized by deposition of contact materials to a non-conductive layer **13 (FIG. 6)** for grounding during the measurement and verifying the performance of conventional MOSFET.

To avoid the diffusion of the solution to bonding wires and metal pads in source/drain regions, the following encapsulation procedure is provided:

(a) Packaging the sensor chip on a plastic (DIP) and bonding of the device by metal wires;

(b) Deposition of a protecting layer on the top of the chip. The protecting layer covers the sensor and bonding wires. Ideally the protecting layer may be an epoxy, which is not etched chemically during the binding of the linker **14,** attaching of chemical/biological receptor molecules **15** on the linker and anchoring of chemical/biological molecule with its complement **16 (FIG 5).**

(c) Opening of a window **17 (FIG. 5)** for the detection surface by an appropriate method. In this case, only the nanoscale electrode array gate is exposed.

All the processes for the manufacturing of the devices, compatible with CMOS technology, are similar to the fabrication processes of the conventional MOSFET except the fact that the blanket metal gate is replaced by a nanoscale electrode array. This is one of the advantages of some embodiments of the present invention.

Using mix&match (UV-lithography and nanoimprint) lithography and introducing source/drain silicidation engineering can enable larger scale production output of the devices and lower manufacturing costs.

Many IDA-gate MOSFET sensor devices may be integrated on one chip to provide selective multiplexed detection of chemical/biological molecules.

As shown in FIG. 7A, 7B, 7C, a linker **14 (FIG. 7A)** is bound selectively on the surface and sidewalls of an IDA-gate **6,7 (FIG. 7A),** then chemical/biological receptor molecules **15 (FIG. 7B)** are attached with the linker **14 (FIG. 7B),** finally a specific chemical/biological molecule which is to be detected **16 (FIG. 7C)** reacts with its complement **15 (FIG. 7C).** Methods to graft receptor molecules to surfaces by appropriate linkers are known and can be found in "Immobilized biomolecules in analysis. A practical approach." [Cass T., Ligler F.S. Eds, Oxford University Press(1998).]. Alternative coupling methods of linker and receptor molecules are also described in "Bioconjugate Techniques", G. T. Hermanson, Academic Press (1996). The linker **14** can be alkylthiol or arylthiol derivatives, alkylsilane derivatives or alkyl carboxylates derivatives which are suitable molecules for selective binding to gold IDA. Preferably, the linker **14** is an alkylthiol or arylthiol derivatives of general formula (I)

$$HS\left[-A-\right]_{n}R \qquad (I)$$

wherein n is an integer, A is hydrocarbon chains which may optionally be perfluorinated, interrupted by hetero atoms or any chemical functionality of interest (sulfones, urea, lactam, etc) and which are preferably non-branched, and wherein R is alkyl, sulfonate functional group, $CO_2H$ and activated forms thereof such as N-hydroxysuccinimidyl ester, $CH_2$-$NH_2$ and activated derivatives such as N-maleimide, $CH_2OH$ and activated forms such as tosylates, $CH_2SH$ and activated forms such as dithiane derivatives, Aryl-$N_3$, $CH_2N=C=O$, $CH_2N=C=S$, nitrile group, nitro group, aldehyde, hydrazine, epoxy, vinyl group or $CO_2R'$ wherein R' is an alkyl group. More preferably, the linker **14** is an alkylthiol or arylthiol derivatives of general formula (I) wherein n is between 5 and 20 and R is $CO_2H$ and activated forms thereof such as N-hydroxysuccinimidyl ester.

These self-assembled monolayers can then be activated by using a carbodiimide reaction and will undergo nucleophilic substitution in the presence the chemical/biological receptor molecules **15.** In a preferred embodiment, the chemical/biological receptor molecules **15** can be a protein having a surface exposed primary amine residues. More preferably, the chemical/biological receptor molecules **15** can be antibodies, antigens, DNA or immunosuppressive protein..

Embodiments of the present invention can offer the ability for detecting quantitatively the presence of chemical/biological molecules, particularly the infection of chemical/biological molecule **16** with its complement **15,** such as protein and DNA.

Before the binding of the linker, a preliminary subthreshold characteristics may be carried out. The subthreshold characteristics may be $I_{ds}$-$V_{gs}$ curve and $I_{ds}$-$V_{ds}$ family curves to obtain more information. The electric measurement may be carried out between both combs of the IDA-gate to obtain capacitance information.

After the attachment of chemical/biological molecules on the linker, the subthreshold characteristics may be measured again. These subthreshold characteristics may be compared with the preliminary subthreshold characteristics.

After the reaction of specific chemical/biological analyte molecule with its complement, the subthreshold characteristics may be further measured. This subthreshold characteristics may be compared with the subthreshold characteristics during the attachment of chemical/biological receptor molecules on the linker.

The embodiments described show an IDA-gate MOSFET sensor which has a N-channel and works on inversion mode, but the present invention is not limited thereto. The conventional N-channel MOSFET is composed of N-type source and drain regions connected by a P-type silicon channel coupled capacitively with a blanket metal/polysilicon gate. The IDA-gate MOSFET sensor may be made by replacing the blanket gate with a nanoscale electrode array. A cross-section of the IDA-gate MOSFET sensor device can be seen in **FIG. 8.** When a bias $V_{gs}$ is applied to one **6 (FIG. 8)** of combs in the array while the other **7 (FIG. 8)** is floating, it is analogous to applying a periodic gate voltage to the device. The current drive is lower in this device than that in the conventional MOSFET due to the presence of a high resistance region **($R_{12}$)** underneath the floating comb and the spacing as shown in **FIG. 8.**

In the equivalent circuit as shown in **FIG. 9,** transistor $\mathbf{T_{11}}$ represents the region covered by the finger **6,** while transistor $T_{12}$ is composed of the region covered by the finger **7** and the spacing region. Two transistors are coupled through a coupling capacitance $C_c$.

The influence of the coupling capacitance can be considered in transistor $T_{12}$, the equivalent circuit can be further represented as a series association of two transistors as shown in **FIG. 10B.** The gate capacitance of transistor $T_{12}$ can be composed of an equivalent capacitance $C_{eq}$, including the normal gate oxide capacitance $C_{ox}$, the capacitance between two fingers $C_{fi}$ and the fringing capacitance $C_{fr}$ between the finger sidewall and oxide surface **(see FIG. 10A).** The equivalent capacitance $C_{eq}$ is given by:

$$C_{eq} = \frac{C_{ox}(C_{fi} + C_{fr})}{C_{ox} + C_{fi} + C_{fr}} \qquad (1)$$

The general expression for the drain current of the conventional MOSFET is:

$$I_d = C_{ox}\mu \frac{W}{L}\left[(V_{gs} - V_t)V_{ds} - \frac{1}{2}V_{ds}^{\ 2}\right] \qquad (2)$$

Where $I_d$ is drain current in the channel, $C_{ox}$ is the gate oxide capacity per unit area, $\mu$ is the electron mobility in the channel, $W$ and $L$ the width and the length of the channel, respectively, $V_{gs}$ is a bias applied to the gate, $V_t$ is the threshold voltage, $V_{ds}$ a voltage between drain and source.

The general expression for the drain current of the IDA-gate MOSFET sensor is the same as Eq. (2) except the fact that the $C_{ox}$ is replaced by $C_{eq}$ in Eq. 1.

The bare IDA-gate MOSFET sensor has a smaller $C_{eq}$ due to small dielectric constant (1 in air). Therefore, drain current is lower, e. g. current does not flow easily between source and drain since an electron-poor layer (high resistance region) appears at the interface underneath the floating comb and the spacing. When the nanoscale electrode array is submersed in a solution containing chemical/biological molecules, a layer of chemical/biological ions/molecules is adsorbed on the array surface. As a result, the equivalent capacitance $C_{eq}$ becomes larger, which is associated with the facts that the dielectric constant of organics is larger than the dielectric constant of vacuum and that the equivalent thickness of the equivalent capacitance is reduced after the binding of molecule layer. Consequently, drain current is increased. In this case, an electron-rich layer forms at the interface underneath the floating comb and the spacing and current can now flow more easily. In other words, for a given gate voltage, the drain current will be a function of the chemical/biological molecule layer thickness and molecule concentration.

It is worth noting that the increase of the drain current caused by the presence of chemical/biological molecules

may be amplified by increasing gate voltage through Eq. (2). It will deform if a signal amplifier is added to a capacitive sensor. This makes the sensitivity of the sensor higher in embodiments of the present invention.

After the binding of chemical/biological receptor molecules and the anchoring of specific chemical/biological molecule/material with its complement, the measurements of the subthreshold characteristics can be not only carried out in a solution but also in air. This makes encapsulation become simpler and easier, which is another advantage of embodiments of the present invention.

**EXAMPLE**

Materials and Process:

A commercially available P-type SOI wafer having a resistivity of 15-25 Ω.cm was used as the starting substrate according to this embodiment of the present invention. Silicon overlay is about 100 nm. A thin pad oxide was deposited on the wafer in order to protect the silicon surface from implantation damage. For a N-type inversion MOSFET, the wafer was implanted by boron with an energy of 20 KeV and a dose of 4.5 $10^{11}$ $cm^{-2}$.

The sensor layout was transferred to the wafer using UV-lithography. Typically, the width of the channel is 30 μm. Reactive ion etching (RIE) was used to pattern the oxide and the silicon overlay. The source/drain regions were defined by UV-lithography and doped by arsenic with an energy of 40 KeV and a dose of 5 $10^{15}$ $cm^{-2}$.

After removing the damaged oxide, a high quality oxide having about 20 nm is grown as the insulating layer. The growing temperature and time of $SiO_2$ were 950°C and 27 min respectively.

Then the layout of the nanoscale interdigitated electrode array was transferred to PMMA by e-beam lithography. The array covers a detection area of 30 X 25 $\mu m^2$. Different finger width and spacing (50 nm, 80 nm, 100 nm and 150 nm) were designed. A stack of 3 nm Ti and 20 nm Au was deposited on the PMMA. The Ti improves the adhesion of Au to oxide. Lift-off was carried out by sonication in hot acetone.

The connection windows were opened to deposit a layer of gold for metal pads. Finally, the back gate was made by depositing a layer of gold.

The chip was packaged on a 40-pin plastic (DIP) and the device was bonded with external electrode by gold wires. A layer of PMMA covered the device and gold wires to protect them from being chemically etched and damaged during the binding of the thiol linker onto the surface of IDA-gate, the attachment of antigen (IRIS) on the linker, and the reaction of antibody (anti-IRIS) with its complement (IRIS). A window was opened to expose the detection surface in a solution.

Measurements and results:

A fabricated IDA-gate MOSFET sensor has a total area of the device is 860 x 860 $\mu m^2$, while the detection area is only 30 x 25 $\mu m^2$. Two source pads, two drain pads and two combs were connected to the external electrodes for electrical characterization and measurement.

The measurement was performed using an HP4156 semiconductor parameter analyzer. The back gate was connected to the ground. All the measurements were carried out in the same conditions (in air, in the dark and in a metallic cage).

Before the binding of the thiol linker on the surface of the IDA-gate, a preliminary measurement of subthreshold characteristics was carried out. The preliminary subthreshold characteristics would be compared with the subthreshold characteristics during the binding of the thiol linker, the attaching of the antigen to the thiol linker and the reaction of antibody to its complementary antigen.

The sensor surface was cleaned in ethanol for 15 min and in hexane for 15 min. After drying under nitrogen, the sensor was immediately immersed in the thiol solution for 24 h at room temperature, followed by rinsing off the residual thiol molecules with ethanol.

After the binding of the thiol linker, a measurement of the subthreshold characteristics of the sensor was carried out further in air. The subthreshold characteristics would be compared with the subthreshold characteristics during the attaching of the antigen to the thiol linker and the reaction of antibody to its complement or with the preliminary subthreshold characteristics.

To covalently immobilize the antigen IRIS on the IDA-gate, the well known EDC-NHS coupling chemistry was used. Activated NHS-ester groups were so created on the IDA-gate surface.

Then, the antigen (IRIS) was immobilized on the surface of the IDA-gate for 1 h by reaction of the amine groups present at the surface of the protein with the NHS-ester groups present at the IDA-gate surface to form amide groups. The measurement of subthreshold characteristics of the sensor was carried out in air. The measurement of subthreshold characteristics would be compared with the measurement of subthreshold characteristics during the reaction of antibody to its complement or with the preliminary measurement of subthreshold characteristics and the measurement of subthreshold characteristics after the binding of thiol linker.

Finally, the antibody (anti-IRIS) was put into contact with the antigen for 1 h. The measurement of the subthreshold characteristics of the sensor was carried out again in air. The measurement of subthreshold characteristics

would be compared with all the subthreshold characteristics measurements obtained previously.

**Fig. 11** gives subthreshold characteristics ($I_{ds}$ - $V_{gs}$ curves) of a fabricated IDA-gate MOSFET sensor for the bare device; after the binding of thiol linker ; after attaching the antigen (IRIS) to the thiol linker and the reaction of antibody (anti-IRIS) with its complementary antigen IRIS, successively. It can be seen that when $V_{gs}$ > 1 V, for a given gate voltage, the drain current is increased with the increase of the organic molecule layer thickness. The increase of the drain current is caused by the increase of the equivalent permittivity $\varepsilon_{eq}$ and the decrease of the equivalent thickness $t_{eq}$ in the equivalent capacitance $C_{eq}$.

It is noted that the change in drain current may be amplified by increasing the gate voltage as shown in **FIG. 11.** This is one remarkable advantage of embodiments of the present invention. In fact, the IDA-gate MOSFET sensor itself is an amplifier, which may amplify the electrical signal variation due to the presence of antigen or the infection of antigen and antibody. This makes the sensitivity of the sensor open to improvement by adjusting the gate voltage.

It can be seen that the presence of IRIS and the infection of IRIS and anti-IRIS are monitored directly by the drain current change rather than by the labels. At least some embodiments of the present invention thus have the advantages of label-free and direct analysis.

It is also noted that, only 1 μl solution containing antigen or antibody was used in the measurement due to a small detection area of 24 x 30μ$m^2$. The need of a small sample volume is another advantage of some embodiments of the present invention.

After finishing all the measurements, a step of physical characterization was used to observe the infection of anti-IRIS and IRIS. **FIG. 12A, 12B** show a SEM top views of the nanoscale IDA-gate covered by anti-IRIS with concentrations of 0.72 ng/ml and 360 ng/ml, respectively.

The sensitivity of the sensor is defined by ($I_a$-$I_b$)/ $I_b$ at constant gate voltage and drain voltage, where $I_b$ and $I_a$ are the drain currents before and after the infection of IRIS and anti-IRIS, respectively. **FIG. 13** shows the sensitivity of a typical sensor as a function of anti-IRIS concentrations. A nearly linear relationship between the sensitivity and logarithmic value of concentration is found in a range from 0.7 ng/ml to 36 μm/ml. The detection limit of this sensor is lower than 1 ng/ml. The high sensitivity and quantitative detection is a remarkable advantage of some embodiments of the present invention.

In order to make sure this sensor does not react with non-specific anti-IRIS, a non-specific antibody (286F7) with a concentration 40 times higher than specific anti-IRIS was first tested with IRIS. No evident change in drain current was observed. If the concentration which is the same as that of anti-IRIS was used, this change will be become much smaller, even undetectable and ignored. This means that there is no reaction between IRIS and 286F7. The highly selective and specific sensing is another advantage of embodiments of this invention.

It is also noted that the sensitivity also depends on finger width and spacing. **Table 1** summarises the sensitivity of three sensors with different finger width and spacing for detecting the infection of IRIS and anti-IRIS at a concentration of 36 μg/ml.

Table 1

| Name | Finger 1 width (nm) | Finger 2 width (nm) | Finger spacing (nm) | Ion covering area (%) | **Sensitivity** |
|---|---|---|---|---|---|
| Sensor A | 150 | 150 | 100 | 40 | 6% |
| Sensor B | 100 | 100 | 100 | 50 | 60 % |
| Sensor C | 80 | 50 | 80 | 55 | 260 % |

To compare sensor A, and B, they have different finger width (150 nm and 100 nm) and the same spacing (100 nm) which means that their $C_{fi}$ is the same, but the high resistance region is shorter in sensor B (300 nm) than in sensor A (350 nm), resulting in a larger $C_{fr}$ in sensor B than in sensor A. Therefore sensor B exhibits a higher sensitivity (60%). Sensor C has the narrowest spacing (80 nm) and the shortest length of the high resistance region (210 nm) among three devices, it presents highest sensitivity (260%). The size effect offers an interesting possibility for optimizing the sensitivity: it turns out that narrower finger width and spacing offer larger sensitivity.

In order to assess the sensing reproducibility, more than 20 sensors were measured after the presence of IRIS and the infection of IRIS and anti-IRIS. The drain current change has been observed in all the measured sensors. Particularly, the performance of bare sensors with identical finger width and spacing display an excellent uniformity. The good reproducibility of the fabrication is another advantage of at least some embodiments of the present invention. As has been described above a sensor is provided, whose construction is similar to that of a conventional MOSFET, except for the fact that the traditional blanket metal/polysilicon gate is replaced by a nanoscale finger-like (interdigitated or not) electrode array. The sensor can be used to quantitatively detect the presence of chemical/biological molecules.

## Claims

1. A sensor for detecting the presence of chemical/biological molecules, , the sensor being an MOSFET device **(1,2,3,6,7,9,10,12,13),** the gate of the MOSFET device having an electrode in the form of an array of fingers **(6,7),** and on fingers or in spaces between the fingers, the device having chemical/biological receptor molecules (15) suitable for selectively binding with the chemical/biological molecules (16), the sensor also having circuitry **(4,5,11)** for measuring a change in electrical characteristics of the FET caused by the chemical/biological molecules becoming bound to the receptor molecules.

2. The sensor of claim 1, the array of fingers being arranged perpendicular to a channel of the device.

3. The sensor of claim 1 or 2, the array of fingers having a comb of fingers **(6).**

4. The sensor of claim 3, the array of fingers having a second comb of fingers **(7).**

5. The sensor of any preceding claim, the MOSFET comprising an IDA-gate **(6,7)**

6. The sensor of any preceding claim, having a linker (14) on a surface facing the space between fingers, for the attachment of the chemical/biological receptor molecules.

7. The sensor of claim 6, wherein the linker (14) is an alkylthiol, arylthiol, alkylsilane or alkyl carboxylates derivative

8. The sensor of claim 7, wherein the linker (14) is an alkylthiol or arylthiol derivatives of general formula (I)

$$HS-[A]_n-R \quad (I)$$

wherein n is between 5 and 20 and R is $CO_2H$ and activated forms thereof

9. The sensor of any preceding claim having a back gate (12) arranged to ground during the measurement or to tune the detection regime of the gate by shifting the threshold voltage.

10. A method of preparing a sensor for detecting the presence of chemical/biological molecules, the sensor being a MOSFET device **(1,2,3,6,7,9,10,12,13),** the gate of the MOSFET device having an electrode in the form of an array of fingers **(6,7)** the method having the steps of applying on fingers or in spaces between the fingers, chemical/biological receptor molecules (15) suitable for selectively binding with the chemical/biological molecules, to enable sensing by measuring a change in electrical characteristics of the FET caused by the chemical/biological molecules becoming bound to the receptor molecules.

11. The method of claim 10, having the step of applying a linker (14) in the spaces before applying the chemical/biological receptor molecules, to retain the chemical/biological receptor molecules on the device.

12. The method of claim 10 having the preliminary step of measuring the electrical characteristics before and after the application of the chemical/biological receptor molecules.

13. A method of using a sensor for detecting the presence of chemical/biological molecules, the sensor being a MOSFET device **(1,2,3,6,7,9,10,12,13),** the gate of the MOSFET device having an electrode in the form of an array of fingers **(6,7)** and in spaces between the fingers, the device having chemical/biological receptor molecules (15) suitable for selectively binding with the chemical/biological molecules, the method having the steps of exposing the sensor, and of measuring a change in electrical characteristics of the FET caused by the chemical/biological molecules becoming bound to the receptor molecules.

14. The method of claim 13, using a sensor prepared by the steps of any of claims 8 to 10, the sensor being as set out in any of claims 1 to 7.

15. The method of claim 13 or 14, the measuring of electrical characteristics comprising measuring a change in drain

current of the device.

**16.** The method of claim 15, the sensor having two combs, the measuring further comprising measuring a change in capacitance or resistance between the combs.

**17.** A method of manufacturing a sensor for detecting the presence of chemical/biological molecules, the method having the steps of forming a MOSFET device (1,2,3,6,7,9,10,12,13), forming a gate (6,7) having an electrode in the form of an array of fingers (6,7), and in spaces between the fingers, applying chemical/biological receptor molecules (15) suitable for selectively binding with the□chemical/biological molecules, and forming circuitry (4,5,11) for measuring a change in electrical characteristics of the FET caused by the chemical/biological molecules becoming bound to the receptor molecules.

**18.** The method of claim 17 having the step of forming the array in the form of a first and a second comb of fingers.

**19.** The method of claim 17 or 18 having the steps of forming an integrated sensor having many of the FET devices, for detecting many different chemical/biological molecules.

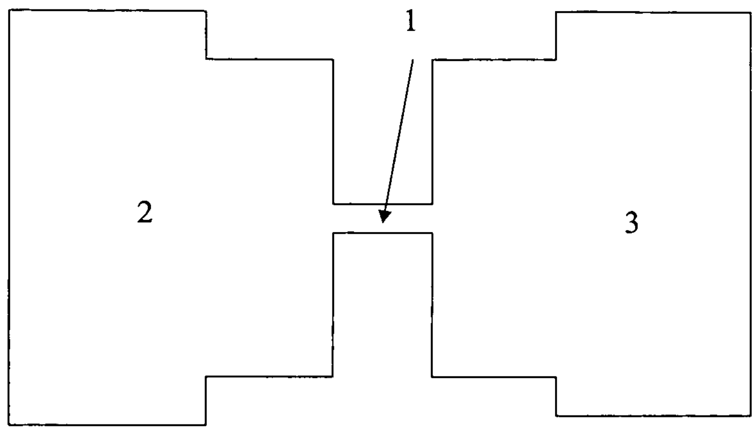


FIG. 1

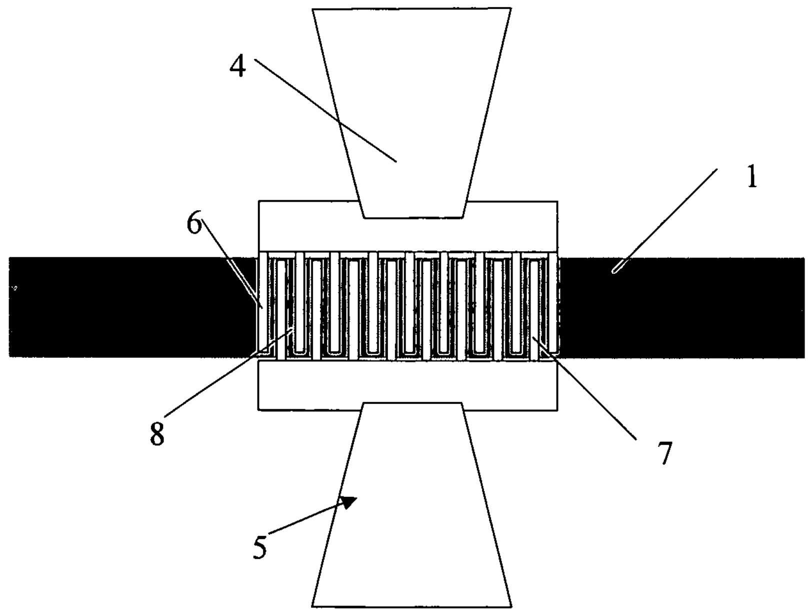


FIG. 2

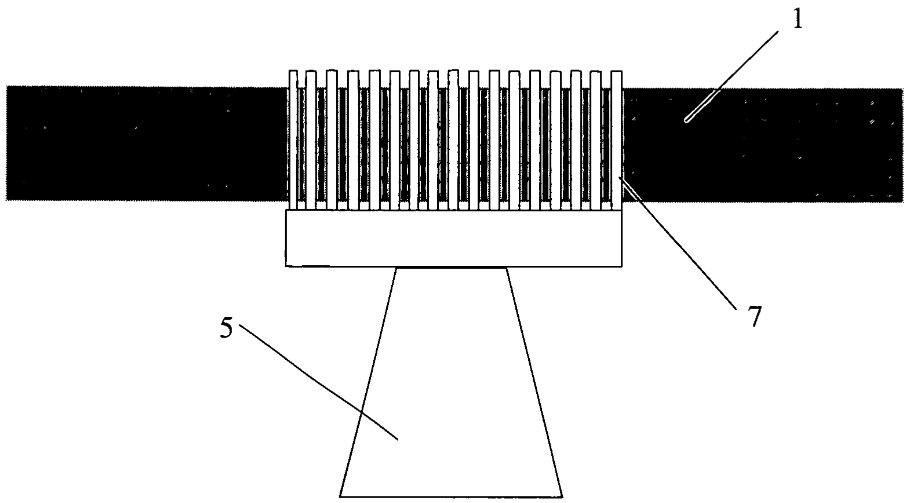


FIG. 3

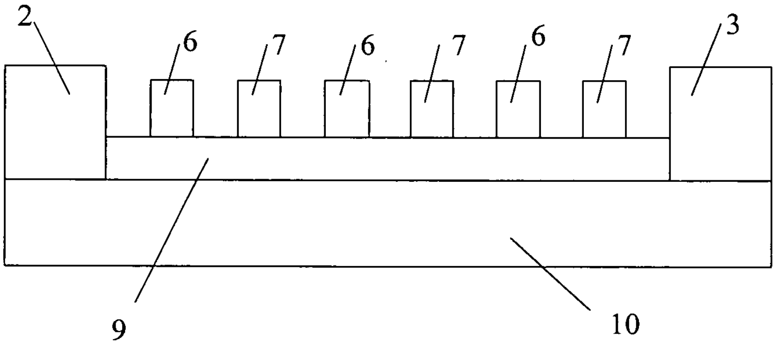


FIG. 4

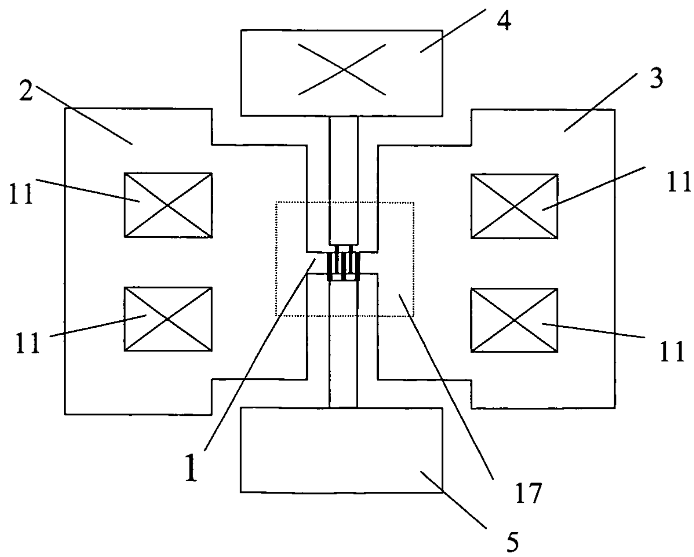


FIG. 5

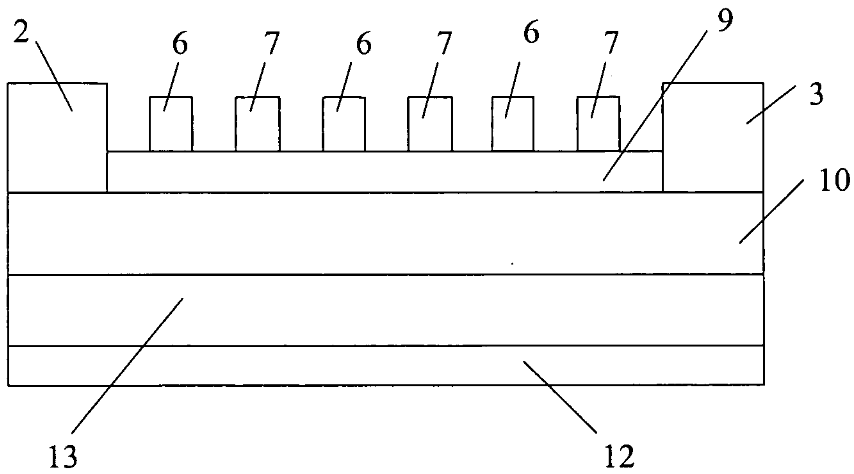


FIG. 6

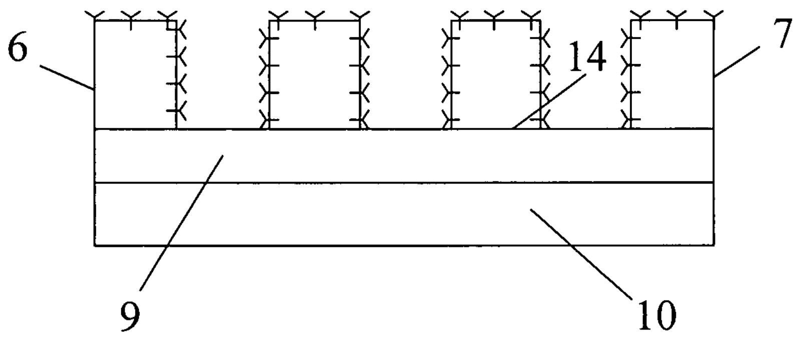


FIG. 7A

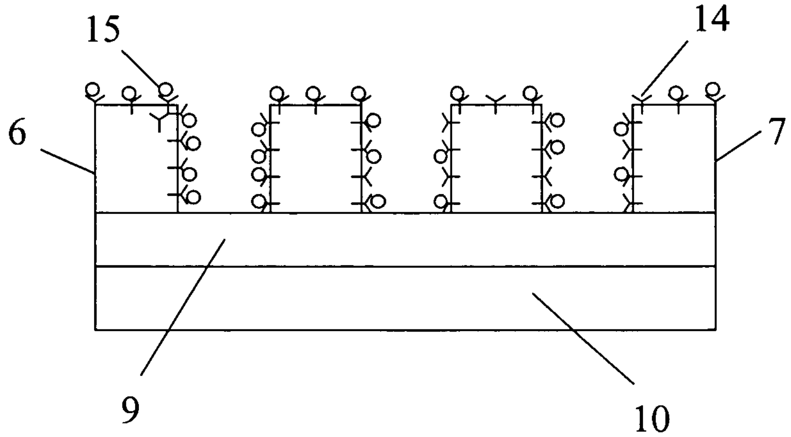


FIG. 7B

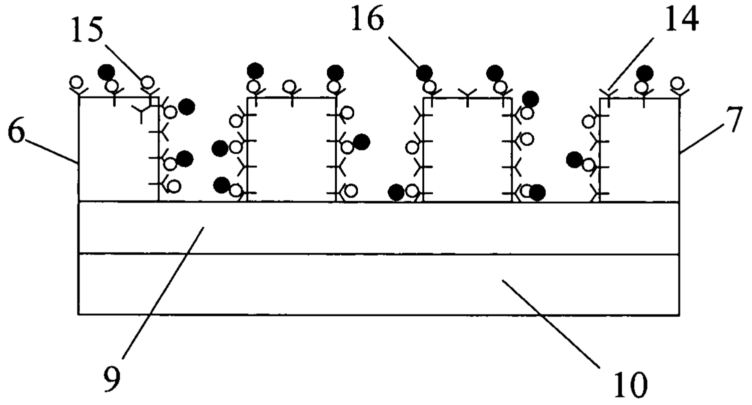


FIG. 7C

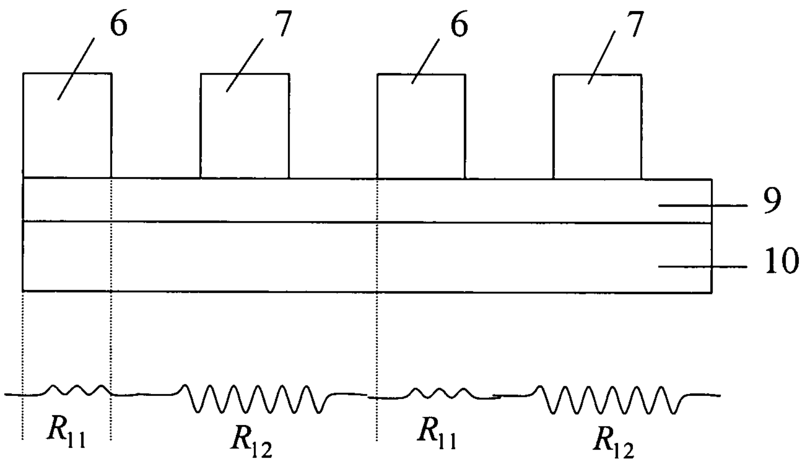


FIG. 8

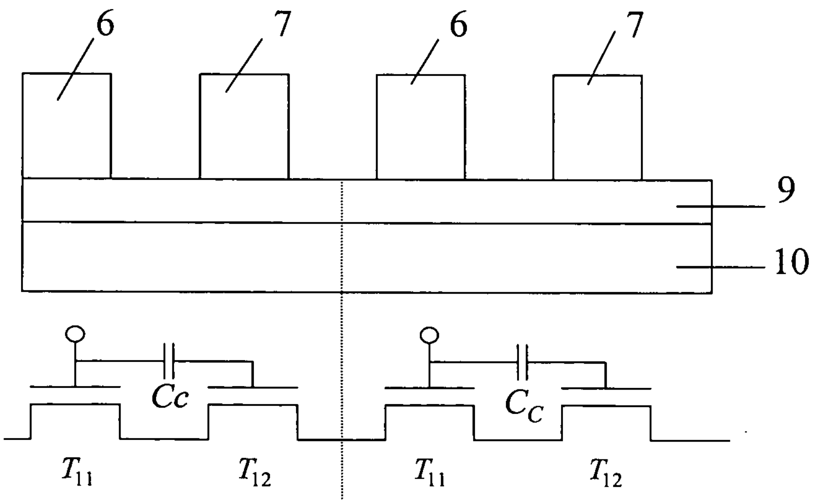


FIG. 9

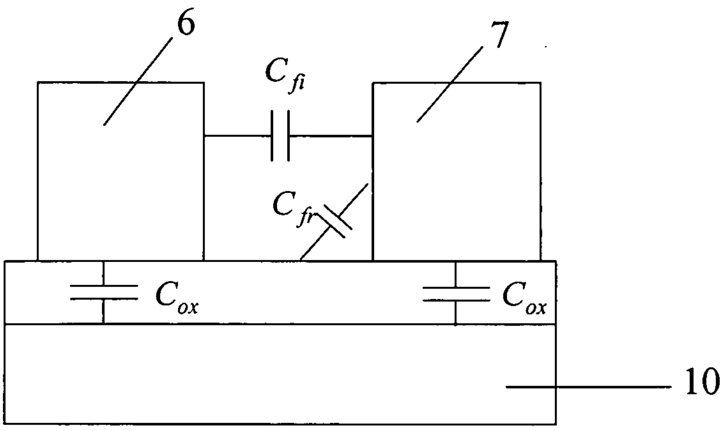


FIG. 10A

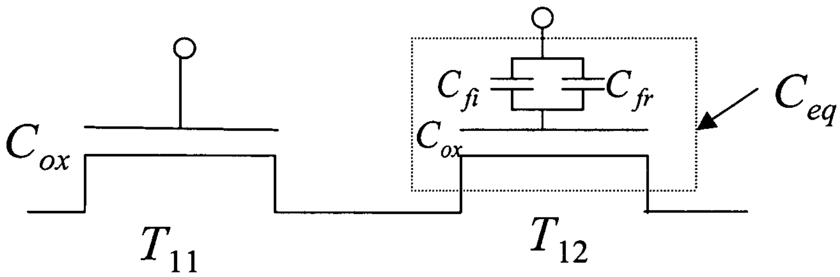


FIG. 10B

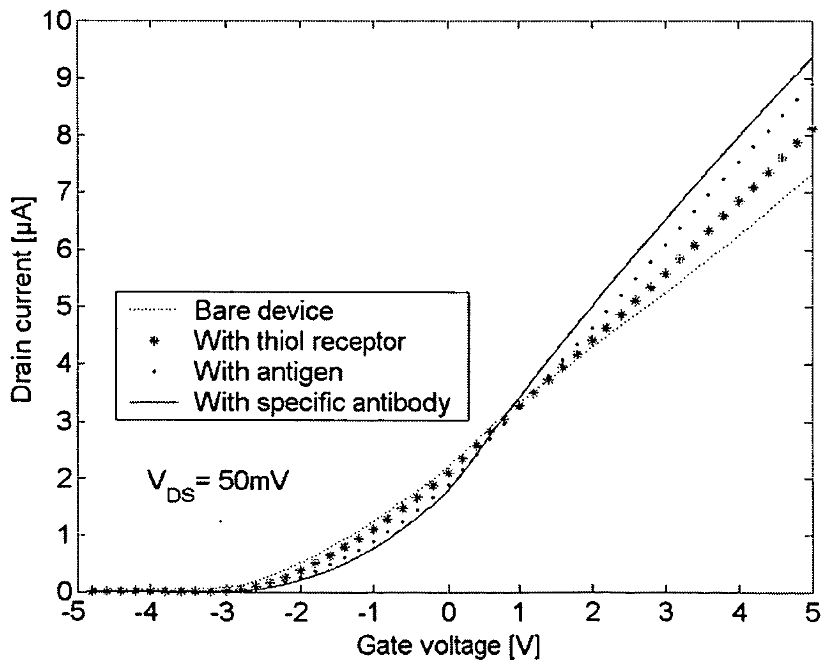


FIG. 11

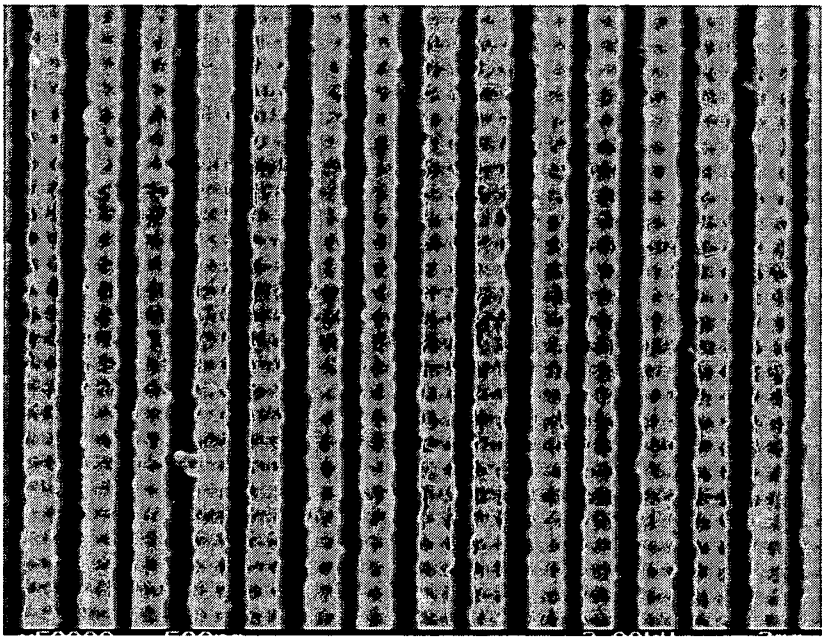

* 50000 500nm 3.00kV

FIG. 12A

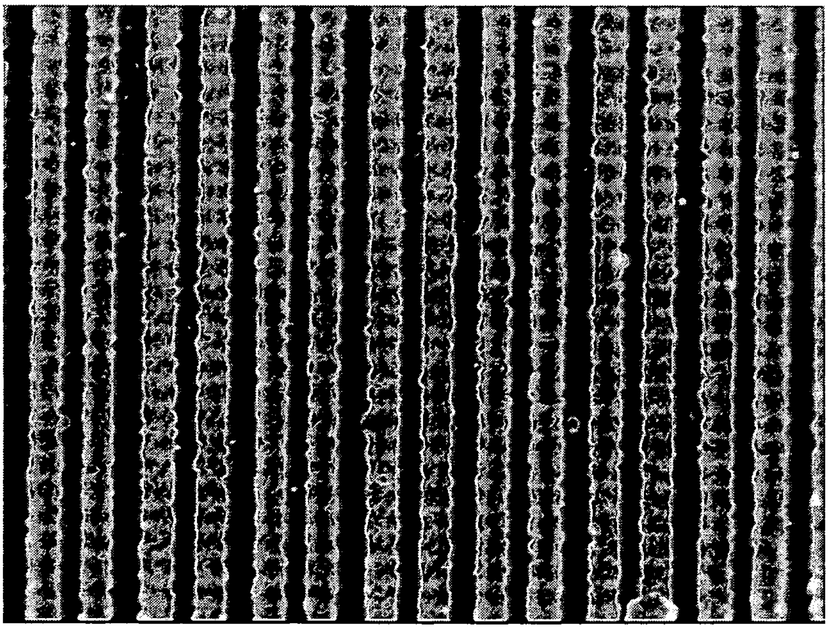

* 50000 500nm 3.00kV

FIG. 12B

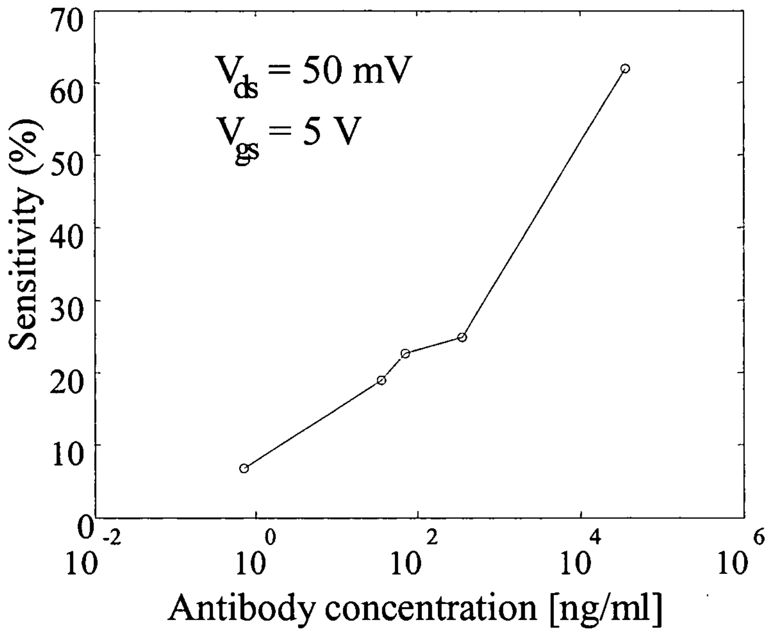

$V_{ds}$ = 50 mV
$V_{gs}$ = 5 V
Sensitivity (%)
0
10
20
30
40
50
60
70
$10^{-2}$
$10^{0}$
$10^{2}$
$10^{4}$
$10^{6}$
Antibody concentration [ng/ml]


**FIG. 13**

European Patent Office

# EUROPEAN SEARCH REPORT

Application Number
EP 07 01 4037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | US 2004/256655 A1 (KAN EDWIN [US] ET AL) 23 December 2004 (2004-12-23)<br>* paragraphs [0005] - [0008], [0017] - [0029] *<br>* abstract; figures 1,2,8 * | 1-8, 10-14, 16,17,19<br>9 | INV. G01N27/414 |
| X | JP 57 161541 A (TOKYO SHIBAURA ELECTRIC CO) 5 October 1982 (1982-10-05)<br>* abstract; figure 1 * | 1-3,5, 10, 13-15,17 | |
| X | US 4 158 807 A (SENTURIA STEPHEN D) 19 June 1979 (1979-06-19)<br>* columns 4-7; figures 1,5,6,21 * | 1-3,5,9, 10, 13-15, 17,19 | |
| X<br><br><br>A | US 2004/110277 A1 (MAEDA HIROSHI [JP]) 10 June 2004 (2004-06-10)<br>* paragraphs [0041] - [0059]; figures 1-7 * | 1,3-6, 10-14, 16-19<br>9 | TECHNICAL FIELDS SEARCHED (IPC)<br>G01N<br>C12Q |
| X | US 5 045 285 A (KOLESAR JR EDWARD S [US]) 3 September 1991 (1991-09-03)<br>* abstract; figures 1,3,9 * | 1-5,10, 11,14, 17-19 | |
| Y | EP 1 736 760 A (HARVARD COLLEGE [US]) 27 December 2006 (2006-12-27)<br>* paragraphs [0077], [0091]; figure 4A * | 9 | |

-/--

The present search report has been drawn up for all claims

| Place of search | Date of completion of the search | Examiner |
|---|---|---|
| Munich | 7 January 2008 | Klein, Marc-Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

European Patent Office

# EUROPEAN SEARCH REPORT

Application Number
EP 07 01 4037

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2006/041224 A (TOKYO SHIBAURA ELECTRIC CO [JP]; O'UCHI SHIN-ICHI [JP]) 20 April 2006 (2006-04-20) * page 20, line 26 - page 27, line 11 * ----- | 9 | |
| A | EP 1 666 873 A (JAPAN SCIENCE & TECH AGENCY [JP]) 7 June 2006 (2006-06-07) * abstract; figure 1 * * paragraphs [0046] - [0055] * ----- | 9 | |
| | | | TECHNICAL FIELDS SEARCHED (IPC) |

The present search report has been drawn up for all claims

4

| Place of search | Date of completion of the search | Examiner |
|---|---|---|
| Munich | 7 January 2008 | Klein, Marc-Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 01 4037

This annex lists the patent family members relating to the patent documents cited in the above-mentioned European search report.
The members are as contained in the European Patent Office EDP file on
The European Patent Office is in no way liable for these particulars which are merely given for the purpose of information.

07-01-2008

| Patent document cited in search report | Publication date | Patent family member(s) | Publication date |
|---|---|---|---|
| US 2004256655 A1 | 23-12-2004 | US 2006038222 A1 | 23-02-2006 |
| JP 57161541 A | 05-10-1982 | NONE | |
| US 4158807 A | 19-06-1979 | CA 1093215 A1 | 06-01-1981 |
| | | GB 1586011 A | 11-03-1981 |
| | | JP 53132981 A | 20-11-1978 |
| US 2004110277 A1 | 10-06-2004 | CN 1602423 A | 30-03-2005 |
| | | EP 1450156 A1 | 25-08-2004 |
| | | WO 03087798 A1 | 23-10-2003 |
| | | TW 594003 B | 21-06-2004 |
| US 5045285 A | 03-09-1991 | NONE | |
| EP 1736760 A | 27-12-2006 | NONE | |
| WO 2006041224 A | 20-04-2006 | CN 101006338 A | 25-07-2007 |
| | | EP 1810015 A1 | 25-07-2007 |
| | | KR 20070045255 A | 02-05-2007 |
| | | US 2006147983 A1 | 06-07-2006 |
| EP 1666873 A | 07-06-2006 | CN 1842704 A | 04-10-2006 |
| | | JP 2005079342 A | 24-03-2005 |
| | | WO 2005022134 A1 | 10-03-2005 |
| | | KR 20060036487 A | 28-04-2006 |
| | | TW 261114 B | 01-09-2006 |
| | | US 2007063304 A1 | 22-03-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20050227373 A1


### Non-patent literature cited in the description


- **CUI et al.** *Science,* 2001, vol. 293, 1289
- **TALIN et al.** Large area, dense silicon nanowire array chemical sensors. *Applied Physics Letters,* 2006, vol. 89, 153102
- **FERNADO et al.** Fabrication of silicon nanowire devices for ultrasensitive, label-free, real-time detection of biological and chemical species. *Nature Protocols,* 2006, vol. 1 (4), 1711
- *Biosensors and Bioelectronics,* 1997, vol. 12, 29
- *Sensors and Actuators B,* 1998, vol. 49, 73
- Immobilized biomolecules in analysis. A practical approach. Oxford University Press, 1998
- **G. T. HERMANSON.** Bioconjugate Techniques. Academic Press, 1996